# EUROPEAN PATENT APPLICATION

(11) **EP 1 634 598 A1**
(43) Date of publication of application: **15.03.2006**
(21) Application number: 04021203.7
(22) Date of filing: 07.09.2004
(51) Int. Cl.: A61K 31/495

(54) **Use of piperazine derivatives and analogues for the manufacture of a medicament for the prophylaxis and/or treatment of disorders of food ingestion**

(71) Applicant: LABORATORIOS DEL DR. ESTEVE, S.A., 08041 Barcelona (ES)
(72) Inventor: Zamanillo-Castanedo, D;Labor. del Dr Esteve S.A., 08041 Barcelona (ES)

(57) **Abstract**

The present invention refers to the use of piperazine derivatives and analogues for the manufacture of a medicament for the prophylaxis and/or treatment of disorders of food ingestion.

## Description

### Field of the invention

The present invention refers to the use of piperazine derivatives and analogues for the manufacture of a medicament for the prophylaxis and/or treatment of disorders of food ingestion.

### Background of the invention

Food ingestion disorders, particularly obesity, are a serious, fast growing threat to the health of humans of all age groups, since they increase the risk of developing other serious, even life-threatening diseases such as diabetes or coronary diseases.

Thus, the object of the present invention was to provide medicaments that comprise compounds which are suitable for the prophylaxis and/or treatment of food-ingestion related disorders.

So, the main object of this invention is the use of a compound according to general formula IA 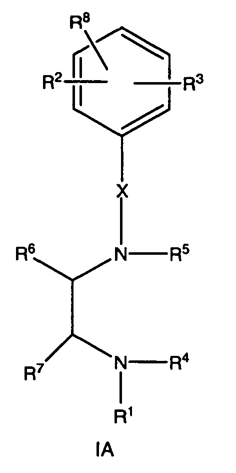 wherein
R¹ is selected from C₁₋₆-Alkyl, saturated or unsaturated, substituted or not substituted, branched or not branched;
R², R³ and R⁸ are independently of each other selected from H; OH, SH, NH₂, C₁₋₆₋Alkyl, saturated or unsaturated, substituted or not substituted, branched or not branched; Halogen; O-C₁₋₆-Alkyl, saturated or unsaturated, substituted or not substituted, branched or not branched; O―C(O)-C₁₋₆-Alkyl, saturated or unsaturated, substituted or not substituted, branched or not branched; C(O)-O-C₁₋₆-Alkyl, saturated or unsaturated, substituted or not substituted, branched or not branched; or NH-C(O)-C₁₋₆-Alkyl, saturated or unsaturated, substituted or not substituted, branched or not branched;
R⁴ and R⁵ are independently of each other selected from H; OH, SH, NH₂, C₁₋₆₋Alkyl, saturated or unsaturated, substituted or not substituted, branched or not branched; Halogen; O-C₁₋₆-Alkyl, saturated or unsaturated, substituted or not substituted, branched or not branched;
or
R⁴ and R⁵ taken together are -(CHR⁹)ₙ- forming a ring
with n is selected from 1, 2 or 3 and
each R⁹ independently selected from H; OH, SH, NH₂, C₁₋₆₋Alkyl, saturated or unsaturated, substituted or not substituted, branched or not branched; Halogen; O-C₁₋₆-Alkyl, saturated or unsaturated, substituted
or not substituted, branched or not branched;
R⁶ and R⁷ are independently of each other selected from H; OH, SH, NH₂, C₁₋₆₋ Alkyl, saturated or unsaturated, substituted or not substituted, branched or not branched; Halogen; or O-C₁₋₆-Alkyl, saturated or unsaturated, substituted or not substituted, branched or not branched;
X is -(CHR¹⁰)ₘ-
with m selected from 0, 1, 2, 3 or 4 and
(if applicable) each R¹⁰ independently selected from H; OH, SH, NH₂, C₁₋₆₋Alkyl, saturated or unsaturated, substituted or not substituted, branched or not branched; Halogen; O-C₁₋₆-Alkyl, saturated or unsaturated, substituted or not substituted, branched or not branched;
optionally in the form of its racemate, pure stereoisomers, especially enantiomers or diastereomers or in the form of mixtures of stereoisomers, especially enantiomers or diastereomers, in any suitable ratio; in the form shown or in form of the acid or base or in form of a salt, especially a physiologically acceptable salt, or in form of a solvate, especially a hydrate;
in the manufacture of a medicament for the prophylaxis and/or treatment of disorders of food ingestion.

The piperazine derivatives and analogues compounds according to formula IA were surprisingly active for the prophylaxis and/or treatment of disorders of food ingestion. Compounds according to formula IA are known in the art and their action on cocaine addiction described in US2003/0171347A1 and WO 99-59409 A1 included here by reference. Especially well known are BD-1063 (1-(3,4-dichlorophenethyl)-4-methylpiperazine/1-[2-(3,4-dichlorophenyl)ethyl]-4-methylpiperazine) and BD-1047 (N1-(3,4-dichlorophenethyl)-N1,N2,N2-trimethylethane-1,2-diamine/ N-[2-(3,4-dichlorophenyl)ethyl]-N-methyl-2-(dimethylamino)-ethylamine) which are both commercially available and popular tool compounds as sigma receptor ligands. As according to US2003/0171347A1 BD-1063 and BD-1047 show no or only slight binding to a selection of other relevant receptors (page 7, table2 and page 8, table 3) it is highly likely that their action as shown in the experimental part is due to their binding to a sigma receptor. Further data can be found in Vilner et al., J. Neurosci. 15, 117-134 (1995).

The synthesis of BD-1063 and compounds structurally related (mostly those covered under formula IB) is described in detail in de Costa et al. (1993), J. Med. Chem. 36(16): 2311-2320 (referred to as compound 4 in Scheme I; p. 2312) included here by reference. The synthesis of BD-1047 is described in detail in de Costa et al. (1992), J. Med Chem. 35, 38-47, as well as WO92/22279 A1 showing compounds related to CNS disorders, where BD-1047 is described as a truncated version (compound 10 in Scheme 2, p.17), both included here by reference. Accordingly the synthesis of the compounds whose use is claimed in this invention is known and the compounds thus available to those skilled in the art, starting from the given information working analogously if necessary.

"Treating" or "treatment" as used in this application are defined as including the treatment of the symptoms - of disorders of food ingestion - as well as treatment of the disease or disease consequences causing the symptoms.

"Prophylaxis" as used in this application is defined as including the prevention or the prophylaxis of the symptoms - of disorders of food ingestion - as well as the prevention or the prophylaxis of the disease or disease consequences causing the symptoms.

"The sigma receptor/s" as used in this application is/are well known and defined using the following citation: This binding site represents a typical protein different from opioid, NMDA, dopaminergic, and other known neurotransmitter or hormone receptor families (G. Ronsisvalle et al. Pure Appl. Chem. 73, 1499-1509 (2001)).

Pharmacological data based on ligand binding studies, anatomical distribution and biochemical features distinguish at least two subtypes of σ receptors ( R. Quiron et al., Trends Pharmacol. Sci. 13, 85-86 (1992); M.L.Leitner, Eur. J. Pharmacol. 259, 65-69 (1994); S.B. Hellewell and W.D. Bowen; Brain Res. 527, 244-253 (1990)) (G. Ronsisvalle et al. Pure Appl. Chem. 73, 1499-1509 (2001)). The protein sequence of sigma receptors (Sigma 1 (σ1) and Sigma 2 (σ2)) is known (e.g. Prasad, P.D. et al., J. Neurochem. 70 (2), 443-451 (1998)) and they show a very high affinity for e.g. pentazocine. Another selective ligand is a compound known as NE-100 (Chaki, S. et al., Eur. J. Pharmacol. 251, R1-R2 (1994)).

In the context of this invention, alkyl and cycloalkyl radicals are understood as meaning saturated and unsaturated (but not aromatic), branched, unbranched and cyclic hydrocarbons, which can be unsubstituted or mono- or polysubstituted. In these radicals, C₁₋₂-alkyl represents C1- or C2-alkyl, C₁₋₃-alkyl represents C1-, C2- or C3-alkyl, C₁₋₄-alkyl represents C1-, C2-, C3- or C4-alkyl, C₁₋₅-alkyl represents C1-, C2-, C3-, C4-, or C5-alkyl, C₁₋₆-alkyl represents C1-, C2-, C3-, C4-, C5- or C6-alkyl, C₁₋₇-alkyl represents C1-, C2-, C3-, C4-, C5-, C6- or C7-alkyl, C₁₋₈-alkyl represents C1-, C2-, C3-, C4-, C5-, C6-, C7- or C8-alkyl, C₁₋₁₀-alkyl represents C1-, C2-, C3-, C4-, C5-, C6-, C7-, C8-, C9- or C10-alkyl and C₁₋₁₈-alkyl represents C1-, C2-, C3-, C4-, C5-, C6-, C7-, C8-, C9-, C10-, C11-, C12-, C13-, C14-, C15-, C16-, C17- or C18-alkyl. Furthermore, C₃₋₄-cycloalkyl represents C3- or C4-cycloalkyl, C₃₋₅- cycloalkyl represents C3-, C4- or C5-cycloalkyl, C₃₋₆-cycloalkyl represents C3-, C4-, C5- or C6-cycloalkyl, C₃₋₇-cycloalkyl represents C3-, C4-, C5-, C6- or C7-cycloalkyl, C₃₋₈-cycloalkyl represents C3-, C4-, C5-, C6-, C7- or C8-cycloalkyl, C₄₋₅-cycloalkyl represents C4- or C5-cycloalkyl, C₄₋₆-cycloalkyl represents C4-, C5- or C6-cycloalkyl, C₄₋₇-cycloalkyl represents C4-, C5-, C6- or C7-cycloalkyl, C₅₋₆-cycloalkyl represents C5- or C6-cycloalkyl and C₅₋₇-cycloalkyl represents C5-, C6- or C7-cycloalkyl. In respect of cycloalkyl, the term also includes saturated cycloalkyls in which one or 2 carbon atoms are replaced by a heteroatom, S, N or O. However, mono- or polyunsaturated, preferably monounsaturated, cycloalkyls without a heteroatom in the ring also in particular fall under the term cycloalkyl as long as the cycloalkyl is not an aromatic system. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, and also adamantyl, (if substituted also CHF₂, CF₃ or CH₂OH) as well as pyrazolinone, oxopyrazolinone, [1,4]-dioxane or dioxolane.

Here, in connection with alkyl and cycloalkyl - unless expressly defined otherwise - the term substituted in the context of this invention is understood as meaning replacement of at least one hydrogen radical by F, Cl, Br, I, NH₂, SH or OH, "polysubstituted" radicals being understood as meaning that the replacement takes effect both on different and on the same atoms several times with the same or different substituents, for example three times on the same C atom, as in the case of CF₃, or at different places, as in the case of -CH(OH)-CH=CH-CHCl₂. Particularly preferred substituents here are F, Cl and OH. In respect of cycloalkyl, the hydrogen radical can also be replaced by OC₁₋₃-alkyl or C₁₋₃-alkyl (in each case mono- or polysubstituted or unsubstituted), in particular methyl, ethyl, n-propyl, i-propyl, CF₃, methoxy or ethoxy.

The term (CH₂)₃₋₆ is to be understood as meaning -CH₂-CH₂-CH₂-, -CH₂-CH₂-CH₂₋CH₂-, -CH₂-CH₂-CH₂-CH₂-CH₂- and -CH₂-CH₂-CH₂-CH₂-CH₂-CH₂-, (CH₂)₁₋₄ is to be understood as meaning -CH₂-, -CH₂-CH₂-, -CH₂-CH₂-CH₂- and -CH₂-CH₂-CH₂-CH₂-, (CH₂)₄₋₅ is to be understood as meaning -CH₂-CH₂-CH₂-CH₂- and -CH₂-CH₂-CH₂₋CH₂-CH₂-, etc.

An aryl radical is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

A heteroaryl radical is understood as meaning heterocyclic ring systems which have at least one unsaturated ring and can contain one or more heteroatoms from the group consisting of nitrogen, oxygen and/or sulfur and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

Here, in connection with aryl and heteroaryl, substituted is understood as meaning substitution of the aryl or heteroaryl by R, OR, a halogen, preferably F and/or Cl, a CF₃, a CN, an NO₂, an NRR, a C₁₋₆-alkyl (saturated), a C₁₋₆₋alkoxy, a C₃₋₈₋cycloalkoxy, a C₃₋₈-cycloalkyl or a C₂₋₆-alkylene.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

In an embodiment of the invention the compound used is a compound according to general formula IA, wherein
R², R³ and R⁸ are independently of each other selected from H; OH, SH, NH₂, C₁₋₆₋Alkyl, saturated or unsaturated, substituted or not substituted, branched or not branched; Halogen; O-C₁₋₆-Alkyl, saturated or unsaturated, substituted or not substituted, branched or not branched.

In another embodiment of the invention the compound used is a compound according to general formula IA, wherein
R⁴ and R⁵ are independently of each other selected from H; OH, NH₂, C₁₋₄₋Alkyl, saturated, not substituted and branched or not branched; Cl, F, Br, I; O-C₁₋₄-Alkyl, saturated, not substituted and branched or not branched;
preferably R⁴ and R⁵ are independently of each other selected from H, CH₃, C₂H₅, C₃H₇, C₄H₉, CF₃, Cl, F, O-CH₃, O-C₂H₅;
most preferably R⁴ and R⁵ are independently of each other selected from H, or CH₃.

In another embodiment of the invention the compound used is a compound according to general formula IA, wherein
R⁴ and R⁵ taken together are -(CHR⁹)ₙ- forming a ring. There it is even preferred if
n is selected from 1 or 2;
preferably n is 2; and/or
each R⁹ is independently selected from H; OH, NH₂, C₁₋₄-Alkyl, saturated, not substituted, branched or not branched; F, Cl, Br, I; O-C₁₋₄-Alkyl, saturated, not substituted, branched or not branched;
preferably each R⁹ is independently selected from H, CH₃, C₂H₅, C₃H₇, C₄H₉, CF₃, Cl, F, O-CH₃, O-C₂H₅;
most preferably R⁹ is H.

In another embodiment of the invention the compound used is a compound according to general formula IA, wherein
R⁶ and R⁷ are independently of each other selected from H; OH, NH₂, C₁₋₄₋Alkyl, saturated, not substituted and branched or not branched; Cl, F, Br, I; O-C₁₋₄-Alkyl, saturated, not substituted and branched or not branched;
preferably R⁶ and R⁷ are independently of each other selected from H, CH₃, C₂H₅, C₃H₇, C₄H₉, CF₃, Cl, F, O-CH₃, O-C₂H₅;
most preferably R⁶ and R⁷ are both H.

In another embodiment of the invention the compound used is a compound according to general formula IA, wherein
X is -(CHR¹⁰)ₘ-
with m selected from 1, 2 or 3,
preferably m is 2;
and/or
each R¹⁰ is independently selected from H; OH, NH₂, C₁₋₄-Alkyl, saturated, not substituted, branched or not branched; F, Cl, Br, I; O-C₁₋₄-Alkyl, saturated, not substituted, branched or not branched;
preferably each R¹⁰ is independently selected from H, CH₃, C₂H₅, C₃H₇, C₄H₉, CF₃, Cl, F, O-CH₃, O-C₂H₅;
most preferably R¹⁰ is H.

In a preferred embodiment of the invention the compound used is a compound according to general formula IA, wherein
R¹ is selected from C₁₋₆-Alkyl, saturated or unsaturated, substituted or not substituted, branched or not branched;
R², R³ and R⁸ are independently of each other selected from H; OH, SH, NH₂, C₁₋₆₋Alkyl, saturated or unsaturated, substituted or not substituted, branched or not branched; Halogen; O-C₁₋₆-Alkyl, saturated or unsaturated, substituted or not substituted, branched or not branched;
R⁴ and R⁵ are independently of each other selected from H, CH₃, C₂H₅, C₃H₇, C₄H₉, CF₃, Cl, F, O-CH₃, O-C₂H₅; preferably H, or CH₃.
or
R⁴ and R⁵ taken together are -(CHR⁹)ₙ- forming a ring,
with n being selected from 1 or 2; preferably 2; and
each R⁹ is independently selected from H, CH₃, C₂H₅, C₃H₇, C₄H₉, CF₃, Cl, F, O-CH₃, O-C₂H₅; preferably H;
R⁶ and R⁷ are independently of each other selected from H, CH₃, C₂H₅, C₃H₇, C₄H₉, CF₃, Cl, F, O-CH₃, O-C₂H₅; preferably H;
X is -(CHR¹⁰)ₘ-
with m being selected from 1, 2 or 3, preferably 2;
and
each R¹⁰ being independently selected from H, CH₃, C₂H₅, C₃H₇, C₄H₉, CF₃, Cl, F, O-CH₃, O-C₂H₅; preferably H.

For this embodiment it is even more preferred if the compound used is a compound according to general formula IA, wherein
R¹ is selected from C₁₋₄-Alkyl, saturated or unsaturated, substituted or not substituted, and branched or not branched;
preferably R¹ is selected from C₁₋₄-Alkyl, saturated, substituted or not substituted, and branched or not branched;
more preferably R¹ is selected from C₁₋₄-Alkyl, saturated, not substituted, and branched or not branched; namely CH₃, C₂H₅, C₃H₇, C₄H₉.

For this embodiment it is also even more preferred if the compound used is a compound according to general formula IA, wherein
R², R³ and R⁸ are independently of each other selected from H; OH, SH, NH₂, C₁₋₄₋Alkyl, saturated or unsaturated, substituted or not substituted, branched or not branched; F, Cl, Br, I; O-C₁₋₄-Alkyl, saturated or unsaturated, substituted or not substituted, branched or not branched;
preferably R², R³ and R⁸ are independently of each other selected from H; OH, NH₂, C₁₋₄₋Alkyl, saturated, substituted or not substituted, and branched or not branched; F, Cl, Br, I; O-C₁₋₄-Alkyl, saturated, not substituted, and branched or not branched;
more preferably R², R³ and R⁸ are independently of each other selected from H, OH, NH₂, CH₃, C₂H₅, C₃H₇, C₄H₉, CF₃, CHF₂, Cl, F, Br, I, O-CH₃, O-C₂H₅, O-C₃H₇, O-C₄H₉;
most preferably R², R³ and R⁸ are independently of each other selected from H, F, Cl and CF₃.

Here it is also preferred if R⁸ is H and R² and R³ are in 3' and 4' position on the phenyl ring.

In another embodiment of the invention the compound used is a compound according to general formula IA selected from
- 1-(3,4-dichlorophenethyl)-4-methylpiperazine or
- N1-(3,4-dichlorophenethyl)-N1,N2,N2-trimethylethane-1,2-diamine
optionally in the form of its racemate, pure stereoisomers, especially enantiomers or diastereomers or in the form of mixtures of stereoisomers, especially enantiomers or diastereomers, in any suitable ratio; in the form described or in form of an acid or base or in form of a salt, especially a physiologically acceptable salt, or in form of a solvate, especially a hydrate.

Another very important and preferred aspect of the current invention is the use of a compound according to general formula IB 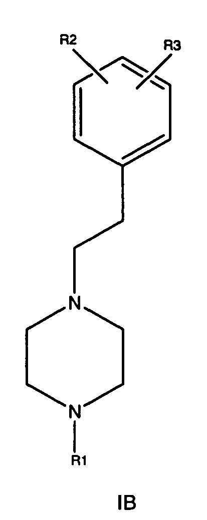 wherein
R1 is selected from C₁₋₆-Alkyl, saturated or unsaturated, substituted or not substituted, branched or not branched;
R2 and R3 are independently of each other selected from H; C₁₋₆₋Alkyl, saturated or unsaturated, substituted or not substituted, branched or not branched; Halogen, O-C₁₋₆-Alkyl, saturated or unsaturated, substituted or not substituted, branched or not branched;
in the manufacture of a medicament for the prophylaxis and/or treatment of disorders of food ingestion.

In a preferred embodiment of the invention the compound used is a compound according to general formula IB, wherein
R¹ is selected from C₁₋₄-Alkyl, saturated or unsaturated, substituted or not substituted, and branched or not branched;
preferably R¹ is selected from C₁₋₄-Alkyl, saturated, substituted or not substituted, and branched or not branched;
more preferably R¹ is selected from C₁₋₄-Alkyl, saturated, not substituted, and branched or not branched; namely CH₃, C₂H₅, C₃H₇, C₄H₉.

In a preferred embodiment of the invention the compound used is a compound according to general formula IB, wherein
R² and R³ are independently of each other selected from H; OH, SH, NH₂, C₁₋₄₋Alkyl, saturated or unsaturated, substituted or not substituted, branched or not branched; F, Cl, Br, I; O-C₁₋₄-Alkyl, saturated or unsaturated, substituted or not substituted, branched or not branched;
preferably R² and R³ are independently of each other selected from H; OH, NH₂, C₁₋₄₋Alkyl, saturated, substituted or not substituted, and branched or not branched; F, Cl, Br, I; O-C₁₋₄-Alkyl, saturated, not substituted, and branched or not branched.

In a preferred embodiment of the invention the compound used is a compound according to general formula IB, wherein
R² and R³ are independently of each other selected from H, OH, NH₂, CH₃, C₂H₅, C₃H₇, C₄H₉, CF₃, CHF₂, Cl, F, Br, I, O-CH₃, O-C₂H₅, O-C₃H₇, O-C₄H₉;
preferably R² and R³ are independently of each other selected from H, F, Cl and CF₃.

Here it is also preferred if R² and R³ are in 3' and 4' position on the phenyl ring.

In a highly preferred embodiment of the invention the compound according to general formula IB used is 1-(3,4-dichlorophenethyl)-4-methylpiperazine, optionally in the form of its racemate, pure stereoisomers, especially enantiomers or diastereomers or in the form of mixtures of stereoisomers, especially enantiomers or diastereomers, in any suitable ratio; in the form described or in form of an acid or base or in form of a salt, especially a physiologically acceptable salt, or in form of a solvate, especially a hydrate.

Unless otherwise stated, the compound of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

"Disorders of food ingestion" are defined according to the invention as including un-or bad regulated appetite, an (from a health aspect or physiologically) undesired body weight (like overweight), obesity, bulimia, anorexia, cachexia or type II diabetes, as well as type II diabetes caused by obesity.

Therefore, in a preferred embodiment of the invention the medicament manufactured according to the invention is intended for the regulation of appetite, for the reduction, increase or maintenance of body weight, for the prophylaxis and/or treatment of obesity, bulimia, anorexia, cachexia or type II diabetes, preferably type II diabetes caused by obesity.

In a highly preferred embodiment of the invention the medicament manufactured according to the invention is intended for the prophylaxis and/or treatment of obesity.

"Obesity" is in the sense of this application is defined according to the web-page of the National Center for Chronic Disease Prevention and Health Promotion of the USA as "an excessively high amount of body fat or adipose tissue in relation to lean body mass. (NRC p114; Stunkard p14) The amount of body fat (or adiposity) includes concern for both the distribution of fat throughout the body and the size of the adipose tissue deposits. Body fat distribution can be estimated by skinfold measures, waist-to-hip circumference ratios, or techniques such as ultrasound, computed tomography, or magnetic resonance imaging."

Overweight refers in the sense of this application according to the web-page of the National Center for Chronic Disease Prevention and Health Promotion of the USA "to increased body weight in relation to height, when compared to some standard of acceptable or desirable weight (NRC p.114; Stunkard p.14)."

Any formulation or pharmaceutical composition according to the invention contains the active ingredient (piperazine derivatives and analogues) as well as optionally at least one auxiliary material and/or additive and/or optionally another active ingredient.

The auxiliary material and/or additive can be specifically selected from conserving agents, emulsifiers and/or carriers for parenteral application. The selection of these auxiliary materials and/or additives and of the amounts to be used depends upon how the pharmaceutical composition is to be applied. Examples include here especially parenteral like intravenous subcutaneous or intramuscular application formulations but which could also be used for other administration routes.

Routes of administration can include inter alia intramuscular injection, intraveneous injection, subcutaneous injection, sublingual, bucal, patch through skin, oral ingestion, implantable osmotic pump, collagen implants, aerosols or suppository.

Solid oral compositions (which are preferred as are liquid ones) may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to the methods well known in normal pharmaceutical practice., in particular with an enteric coating.

The mentioned formulations will be prepared using standard methods such as those described or referred to in the Spanish and US Pharmacopeias and similar reference texts.

Generally an effective administered amount of a compound used according to the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000/mg/kg/day.

Included in this invention are especially also methods of treatments of a patient or a mammal, including men, suffering from disorders of food ingestion using the compounds described as being used according to this invention.

The examples and figures in the following section describing pharmacological trials are merely illustrative and the invention cannot be considered in any way as being restricted to these applications.

### Figures:

Figure 1) refers to example 2 and shows the effect of PRE 084 and BD 1063 on the weight (in weight difference) of chronically treated Obese Rats (DIO) compared to DIO rats treated with Saline (SAL).
Figure 2) refers to example 2 and shows the effect of PRE 084 (DIO-PRE) and BD 1063 (DIO-BD1063) on the blood glucose levels of chronically treated obese rats (DIO) compared to obese rats (DIO) treated with saline (DIO-SALINO) and normal rats treated with saline (SD-SALINO).
Figure 3) refers to example 2 and shows the effect of PRE 084 (DIO-PRE) and BD 1063 (DIO-BD1063) on the blood level of cholesterol of chronically treated obese rats (DIO) compared to obese rats (DIO) treated with saline (DIO-SALINO) and normal rats treated with saline (SD-SALINO).
Figure 4) refers to example 2 and shows the effect of PRE 084 (DIO-PRE) and BD 1063 (DIO-BD1063) on the blood level of Triglycerides (TG) of chronically treated obese rats (DIO) compared to obese rats (DIO) treated with saline (DIO-SALINO) and normal rats treated with saline (SD-SALINO).

### Examples:

### Example 1:

### Example formulation of an parenteral, injectable (im/iv) solution of BD-1063 (1-(3,4-dichlorophenethyl)-4-methylpiperazine)

38.5 g of 3,4-dichlorophenethyl)-4-methylpiperazine were dissolved in 1 I water for injection purposes at ambient temperature and subsequently adjusted to isotonic conditions by addition of anhydrous glucose for injection purposes.

### Example 2:

### Chronical Treatment of Obese Rats (DIO) with PRE 084 and BD 1063:

Obese Rats (DIO) were treated for 9 days intraperitoneally (i.p.) one injection per day with Saline, with BD-1063 at 50 mg/Kg and PRE-084 at 25 mg /Kg, a well known Sigma Inhibitor. During that time each day and at some points during the following 10 days the weight giving the difference in weight (referring to the day of starting the treatment) was determined, as were the blood glucose levels, blood level of cholesterol, blood level of Triglycerides (TG).

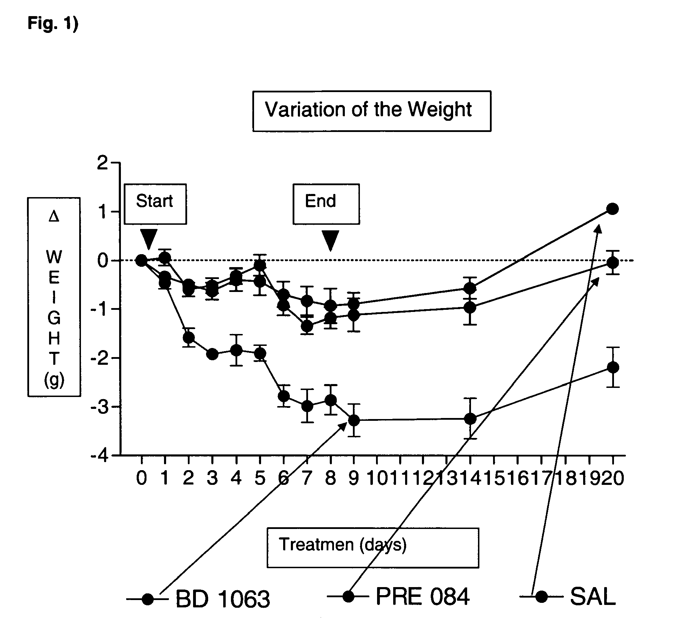

As is clear from these measurements shown in Figures 1 to 4 BD 1063 was highly effective.
Figure 1) shows the effect of PRE 084 and BD 1063 on the weight (in weight difference) of chronically treated Obese Rats (DIO) compared to DIO rats treated with Saline (SAL).

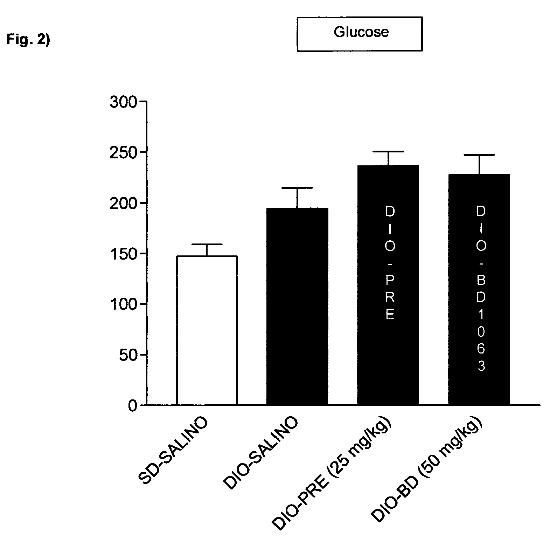

Figure 2) shows the effect of PRE 084 (DIO-PRE) and BD 1063 (DIO-BD1063) on the blood glucose levels of chronically treated obese rats (DIO) compared to obese rats (DIO) treated with saline (DIO-SALINO) and normal rats treated with saline (SD-SALINO).

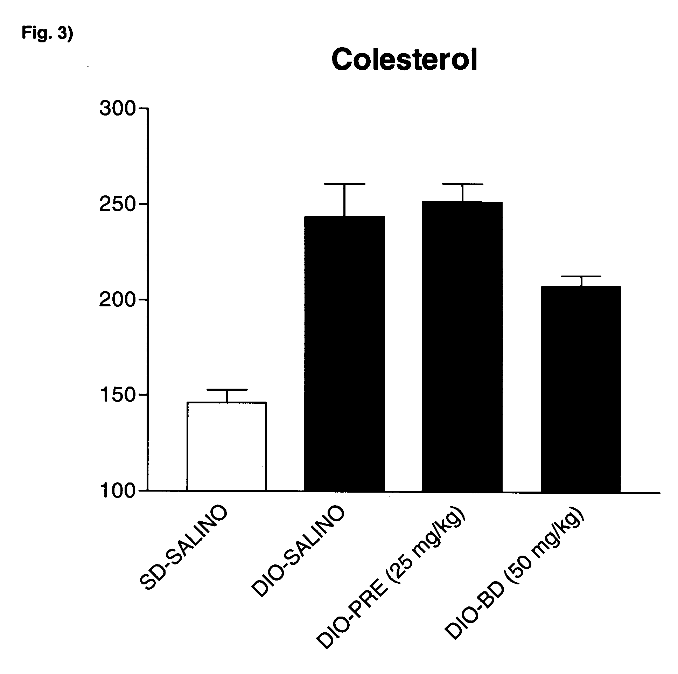

Figure 3) shows the effect of PRE 084 (DIO-PRE) and BD 1063 (DIO-BD1063) on the blood level of cholesterol of chronically treated obese rats (DIO) compared to obese rats (DIO) treated with saline (DIO-SALINO) and normal rats treated with saline (SD-SALINO).

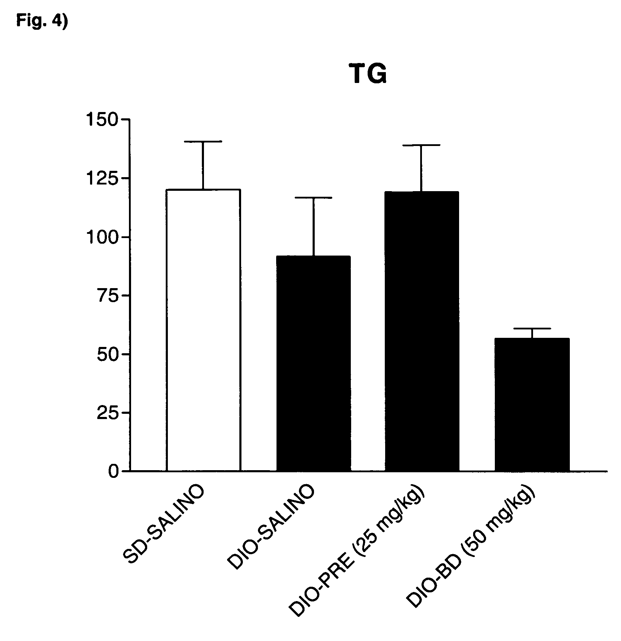

Figure 4) shows the effect of PRE 084 (DIO-PRE) and BD 1063 (DIO-BD1063) on the blood level of Triglycerides (TG) of chronically treated obese rats (DIO) compared to obese rats (DIO) treated with saline (DIO-SALINO) and normal rats treated with saline (SD-SALINO).

## Claims

1. Use of a compound according to general formula IA 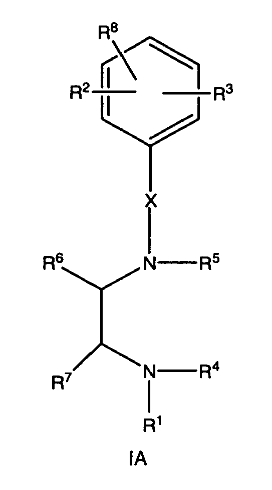
wherein
R¹ is selected from C₁₋₆-Alkyl, saturated or unsaturated, substituted or not substituted, branched or not branched;
R², R³ and R⁸ are independently of each other selected from H; OH, SH, NH₂, C₁₋₆₋Alkyl, saturated or unsaturated, substituted or not substituted, branched or not branched; Halogen; O-C₁₋₆-Alkyl, saturated or unsaturated, substituted or not substituted, branched or not branched; O―C(O)-C₁₋₆-Alkyl, saturated or unsaturated, substituted or not substituted, branched or not branched; C(O)-O-C₁₋₆-Alkyl, saturated or unsaturated, substituted or not substituted, branched or not branched; or NH-C(O)-C₁₋₆-Alkyl, saturated or unsaturated, substituted or not substituted, branched or not branched;
R⁴ and R⁵ are independently of each other selected from H; OH, SH, NH₂, C₁₋₆₋Alkyl, saturated or unsaturated, substituted or not substituted, branched or not branched; Halogen; O-C₁₋₆-Alkyl, saturated or unsaturated, substituted or not substituted, branched or not branched;
or
R⁴ and R⁵ taken together are -(CHR⁹)ₙ- forming a ring
with n is selected from 1, 2 or 3 and
each R⁹ independently selected from H; OH, SH, NH₂, C₁₋₆₋Alkyl, saturated or unsaturated, substituted or not substituted, branched or not branched; Halogen; O-C₁₋₆-Alkyl, saturated or unsaturated, substituted or not substituted, branched or not branched;
R⁶ and R⁷ are independently of each other selected from H; OH, SH, NH₂, C₁₋₆₋Alkyl, saturated or unsaturated, substituted or not substituted, branched or not branched; Halogen; or O-C₁₋₆-Alkyl, saturated or unsaturated, substituted or not substituted, branched or not branched;
X is -(CHR¹⁰)ₘ-
with m selected from 0, 1, 2, 3 or 4 and
(if applicable) each R¹⁰ independently selected from H; OH, SH, NH₂, C₁₋₆₋Alkyl, saturated or unsaturated, substituted or not substituted, branched or not branched; Halogen; O-C₁₋₆-Alkyl, saturated or unsaturated, substituted or not substituted, branched or not branched;
optionally in the form of its racemate, pure stereoisomers, especially enantiomers or diastereomers or in the form of mixtures of stereoisomers, especially enantiomers or diastereomers, in any suitable ratio; in the form shown or in form of the acid or base or in form of a salt, especially a physiologically acceptable salt, or in form of a solvate, especially a hydrate;
in the manufacture of a medicament for the prophylaxis and/or treatment of disorders of food ingestion.

2. Use according to claim 1, in which for the compound according to general formula IA
R², R³ and R⁸ are independently of each other selected from H; OH, SH, NH₂, C₁₋₆₋Alkyl, saturated or unsaturated, substituted or not substituted, branched or not branched; Halogen; O-C₁₋₆-Alkyl, saturated or unsaturated, substituted or not substituted, branched or not branched.

3. Use according to claim 1, in which for the compound according to general formula IA
R⁴ and R⁵ are independently of each other selected from H; OH, NH₂, C₁₋₄₋Alkyl, saturated, not substituted and branched or not branched; Cl, F, Br, I; O-C₁₋₄-Alkyl, saturated, not substituted and branched or not branched;
preferably R⁴ and R⁵ are independently of each other selected from H, CH₃, C₂H₅, C₃H₇, C₄H₉, CF₃, Cl, F, O-CH₃, O-C₂H₅;
most preferably R⁴ and R⁵ are independently of each other selected from H, or CH₃.

4. Use according to claim 1, in which for the compound according to general formula IA
R⁴ and R⁵ taken together are -(CHR⁹)ₙ- forming a ring.

5. Use according to claim 5, in which
n is selected from 1 or 2;
preferably n is 2; and/or
each R⁹ is independently selected from H; OH, NH₂, C₁₋₄-Alkyl, saturated, not substituted, branched or not branched; F, Cl, Br, I; O-C₁₋₄-Alkyl, saturated, not substituted, branched or not branched;
preferably each R⁹ is independently selected from H, CH₃, C₂H₅, C₃H₇, C₄H₉, CF₃, Cl, F, O-CH₃, O-C₂H₅;
most preferably R⁹ is H.

6. Use according to claim 1, in which for the compound according to general formula IA
R⁶ and R⁷ are independently of each other selected from H; OH, NH₂, C₁₋₄₋Alkyl, saturated, not substituted and branched or not branched; CI, F, Br, I; O-C₁₋₄-Alkyl, saturated, not substituted and branched or not branched;
preferably R⁶ and R⁷ are independently of each other selected from H, CH₃, C₂H₅, C₃H₇, C₄H₉, CF₃, Cl, F, O-CH₃, O-C₂H₅;
most preferably R⁶ and R⁷ are both H.

7. Use according to claim 1, in which for the compound according to general formula IA
X is -(CHR¹⁰)ₘ-
with m selected from 1, 2 or 3,
preferably n is 2;
and/or
each R¹⁰ is independently selected from H; OH, NH₂, C₁₋₄₋Alkyl, saturated, not substituted, branched or not branched; F, Cl, Br, I; O-C₁₋₄-Alkyl, saturated, not substituted, branched or not branched;
preferably each R¹⁰ is independently selected from H, CH₃, C₂H₅, C₃H₇, C₄H₉, CF₃, Cl, F, O-CH₃, O-C₂H₅;
most preferably R¹⁰ is H.

8. Use according to claim 1, in which for the compound according to general formula IA
R¹ is selected from C₁₋₆-Alkyl, saturated or unsaturated, substituted or not substituted, branched or not branched;
R², R³ and R⁸ are independently of each other selected from H; OH, SH, NH₂, C₁₋₆₋Alkyl, saturated or unsaturated, substituted or not substituted, branched or not branched; Halogen; O-C₁₋₆-Alkyl, saturated or unsaturated, substituted or not substituted, branched or not branched;
R⁴ and R⁵ are independently of each other selected from H, CH₃, C₂H₅, C₃H₇, C₄H₉, CF₃, Cl, F, O-CH₃, O-C₂H₅; preferably H, or CH₃.
or
R⁴ and R⁵ taken together are -(CHR⁹)ₙ- forming a ring,
with n being selected from 1 or 2; preferably 2; and
each R⁹ is independently selected from H, CH₃, C₂H₅, C₃H₇, C₄H₉, CF₃, Cl, F, O-CH₃, O-C₂H₅; preferably H;
R⁶ and R⁷ are independently of each other selected from H, CH₃, C₂H₅, C₃H₇, C₄H₉, CF₃, Cl, F, O-CH₃, O-C₂H₅; preferably H;
X is -(CHR¹⁰)ₘ-
with m being selected from 1, 2 or 3, preferably 2;
and
each R¹⁰ being independently selected from H, CH₃, C₂H₅, C₃H₇, C₄H₉, CF₃, Cl, F, O-CH₃, O-C₂H₅; preferably H.

9. Use according to claim 8, in which for the compound according to general formula IA
R¹ is selected from C₁₋₄-Alkyl, saturated or unsaturated, substituted or not substituted, and branched or not branched;
preferably R¹ is selected from C₁₋₄-Alkyl, saturated, substituted or not substituted, and branched or not branched;
more preferably R¹ is selected from C₁₋₄-Alkyl, saturated, not substituted, and branched or not branched; namely CH₃, C₂H₅, C₃H₇, C₄H₉.

10. Use according to claims 8 or 9, in which for the compound according to general formula IA
R², R³ and R⁸ are independently of each other selected from H; OH, SH, NH₂, C₁₋₄-Alkyl, saturated or unsaturated, substituted or not substituted, branched or not branched; F, Cl, Br, I; O-C₁₋₄-Alkyl, saturated or unsaturated, substituted or not substituted, branched or not branched;
preferably R², R³ and R⁸ are independently of each other selected from H; OH, NH₂, C₁₋₄₋Alkyl, saturated, substituted or not substituted, and branched or not branched; F, Cl, Br, I; O-C₁₋₄-Alkyl, saturated, not substituted, and branched or not branched;
more preferably R², R³ and R⁸ are independently of each other selected from H, OH, NH₂, CH₃, C₂H₅, C₃H₇, C₄H₉, CF₃, CHF₂, Cl, F, Br, I, O-CH₃, O-C₂H₅, O-C₃H₇, O-C₄H₉;
most preferably R², R³ and R⁸ are independently of each other selected from H, F, Cl and CF₃.

11. Use according to claim 10, in which for the compound according to general formula IA
R⁸ is H and R² and R³ are in 3' and 4' position.

12. Use according to any of claims 1, 8 or 9, in which for the compound according to general formula IA is selected from
• 1-(3,4-dichlorophenethyl)-4-methylpiperazine or
• N1-(3,4-dichlorophenethyl)-N1,N2,N2-trimethylethane-1,2-diamine.

13. Use of a compound according to general formula IB 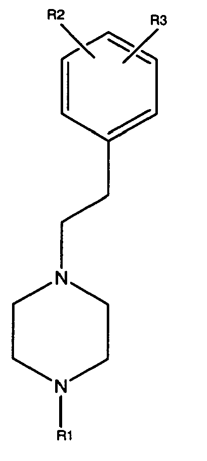
wherein
R1 is selected from C1-6-Alkyl, saturated or unsaturated, substituted or not substituted, branched or not branched;
R2 and R3 are independently of each other selected from H; C₁₋₆₋Alkyl, saturated or unsaturated, substituted or not substituted, branched or not branched; Halogen, O-C₁₋₆-Alkyl, saturated or unsaturated, substituted or not substituted, branched or not branched;
in the manufacture of a medicament for the prophylaxis and/or treatment of disorders of food ingestion.

14. Use according to claim 13, in which for the compound according to general formula IB
R¹ is selected from C₁₋₄-Alkyl, saturated or unsaturated, substituted or not substituted, and branched or not branched;
preferably R¹ is selected from C₁₋₄-Alkyl, saturated, substituted or not substituted, and branched or not branched;
more preferably R¹ is selected from C₁₋₄-Alkyl, saturated, not substituted, and branched or not branched; namely CH₃, C₂H₅, C₃H₇, C₄H₉.

15. Use according to claims 13 or 14, in which for the compound according to general formula IB
R² and R³ are independently of each other selected from H; OH, SH, NH₂, C₁₋₄₋Alkyl, saturated or unsaturated, substituted or not substituted, branched or not branched; F, Cl, Br, I; O-C₁₋₄-Alkyl, saturated or unsaturated, substituted or not substituted, branched or not branched;
preferably R² and R³ are independently of each other selected from H; OH, NH₂, C₁₋₄₋Alkyl, saturated, substituted or not substituted, and branched or not branched; F, Cl, Br, I; O-C₁₋₄-Alkyl, saturated, not substituted, and branched or not branched.

16. Use according to claim 15, in which for the compound according to general formula IB
R² and R³ are independently of each other selected from H, OH, NH₂, CH₃, C₂H₅, C₃H₇, C₄H₉, CF₃, CHF₂, Cl, F, Br, I, O-CH₃, O-C₂H₅, O-C₃H₇, O-C₄H₉;
preferably R² and R³ are independently of each other selected from H, F, Cl and CF₃
and/or are in 3' and 4' position respectively.

17. Use according to any of claims 12 to 16, in which for the compound according to general formula IB is 1-(3,4-dichlorophenethyl)-4-methylpiperazine.

18. Use according to any of claims 1 to 17 for the manufacture of a medicament for the regulation of appetite, for the reduction, increase or maintenance of body weight, for the prophylaxis and/or treatment of obesity, bulimia, anorexia, cachexia or type II diabetes, preferably type II diabetes caused by obesity.

19. Use according to claim 18 for the manufacture of a medicament for the prophylaxis and/or treatment of obesity.
